# EUROPEAN PATENT APPLICATION

(11) **EP 3 231 431 A1**
(43) Date of publication of application: **18.10.2017**
(21) Application number: 15868498.5
(22) Date of filing: 11.12.2015
(51) Int. Cl.: A61K 31/551, A61K 9/08, A61K 31/352, A61K 31/435, A61K 31/5377, A61K 47/22, A61K 47/32, A61P 27/02, A61P 27/06, A61P 43/00

(54) **AQUEOUS COMPOSITION**

(30) Priority: 12.12.2014 JP 2014251695; 12.12.2014 JP 2014251697; 12.12.2014 JP 2014252192
(71) Applicant: Kowa Company, Ltd., Nagoya-shi, Aichi-ken 460-8625 (JP)
(72) Inventor: SUZUKI, Yuuki, Fuji-shi Shizuoka 417-8650 (JP); SAWAI, Isamu, Fuji-shi Shizuoka 417-8650 (JP); ODA, Hiroshi, Fuji-shi Shizuoka 417-8650 (JP)
(74) Representative: Schiener, Jens
(86) International application number: PCT/JP2015/084804
(87) International publication number: WO 2016/093346

(57) **Abstract**

A technique is provided for reducing the discoloration of an aqueous composition containing a halogenated isoquinoline derivative during high-temperature preservation. An aqueous composition comprising a compound represented by Formula (1): 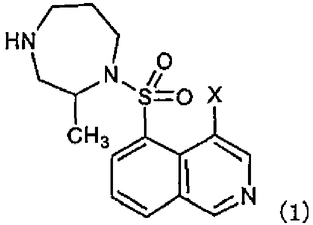 wherein X represents a halogen atom,
or a salt thereof, or a solvate of the compound or the salt thereof, and a β blocker.

## Description

### [Technical Field]

The present invention relates to an aqueous composition and the like.

### [Background Art]

It is known that halogenated isoquinoline derivatives such as ripasudil (chemical name: 4-fluoro-5-{[(2S)-2-methyl-1,4-diazepan-1-yl]sulfonyl}isoquinoline) represented by the following structural formula: 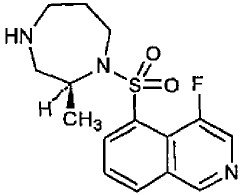 and 4-bromo-5-{[(2S)-2-methyl-1,4-diazepan-1-yl]sulfonyl}isoquinoline represented by the following structural formula: 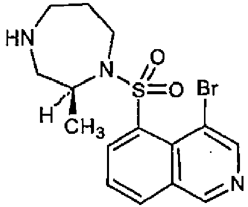 have pharmacological action such as Rho kinase inhibitory action (Patent Literatures 1 and 2, for example), and thus, are usable for the prevention or treatment of eye diseases. Specifically, these halogenated isoquinoline derivatives have been reported to be useful, for example, for the prevention or treatment of ocular hypertension, glaucoma, and the like (Patent Literature 3, for example), or for the prevention or treatment of ocular fundus diseases such as age-related macular degeneration and the like (Patent Literature 4, for example).

Hence, it is extremely useful to establish a technique for producing stable preparations of these halogenated isoquinoline derivatives as ophthalmic agents, for example.

Patent Literature 5 describes a combination of ripasudil ((S)-(-)-1-(4-fluoro-5-isoquinolinesulfonyl)-2-methyl-1,4-homopiperazine) or a salt thereof, and nipradilol as a β blocker. However, Patent Literature 5 only discloses that a solution containing 1% ripasudil and an eye drop containing 0.25% nipradilol were sequentially instilled in one eye of a rabbit, and does not disclose at all an aqueous composition containing both of these components, storing such a composition in a container, the preservation stability over time, and the like.

### [Citation List]

### [Patent Literature]

[Patent Literature 1] JP-B-4212149
[Patent Literature 2] WO2006/115244
[Patent Literature 3] WO2006/068208
[Patent Literature 4] JP-B-5557408
[Patent Literature 5] JP-A-2006-348028

### [Summary of Invention]

### [Technical Problem]

An ophthalmic agent is generally a composition containing water (aqueous composition). To produce a preparation of a halogenated isoquinoline derivative, ripasudil, as an ophthalmic agent or the like, the present inventors initially prepared an aqueous composition containing ripasudil and the preservation stability was investigated, and as a result the aqueous composition was revealed to be disadvantageously discolored over time during high-temperature preservation.

Accordingly, it is an object of the present invention to provide a technique for reducing the discoloration of an aqueous composition containing a halogenated isoquinoline derivative during high-temperature preservation.

### [Solution to Problem]

Thus, the present inventors conducted extensive research to solve the above-described problem, and found that further incorporation of a β blocker such as carteolol and nipradilol in an aqueous composition containing a halogenated isoquinoline derivative such as ripasudil can reduce the discoloration during high-temperature preservation, thus completing the present invention.

In summary, the present invention provides an aqueous composition comprising a compound represented by Formula (1): 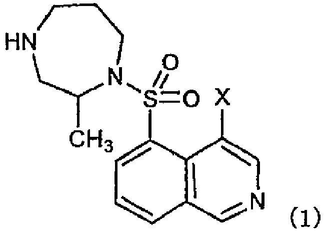 wherein X represents a halogen atom,
or a salt thereof, or a solvate of the compound or the salt thereof, and a β blocker.

Further, the present invention provides a method for reducing discoloration of an aqueous composition, the method comprising the step of incorporating a β blocker in an aqueous composition comprising a compound represented by Formula (1) or a salt thereof, or a solvate of the compound or the salt thereof.

### [Effects of Invention]

In accordance with the present invention, the discoloration of an aqueous composition containing a halogenated isoquinoline derivative such as ripasudil during high-temperature preservation can be reduced.

### [Description of Embodiments]

The present specification discloses, although is in no way limited to, the following embodiments of invention, by way of example.
[1] An aqueous composition comprising a compound represented by Formula (1): 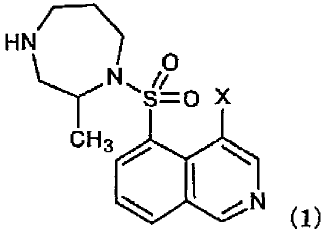 wherein X represents a halogen atom,
   or a salt thereof, or a solvate of the compound or the salt thereof, and a β blocker.
[2] The aqueous composition according to [1], wherein the compound represented by Formula (1) is ripasudil.
[3] The aqueous composition according to [1] or [2], wherein the β blocker is one or more selected from the group consisting of carteolol, timolol, nipradilol, betaxolol, levobunolol, befunolol, metipranolol, a salt of carteolol, a salt of timolol, a salt of nipradilol, a salt of betaxolol, a salt of levobunolol, a salt of befunolol, a salt of metipranolol, a solvate of carteolol or the salt thereof, a solvate of timolol or the salt thereof, a solvate of nipradilol or the salt thereof, a solvate of betaxolol or the salt thereof, a solvate of levobunolol or the salt thereof, a solvate of befunolol or the salt thereof, and a solvate of metipranolol or the salt thereof.
[4] The aqueous composition according to [1] or [2], wherein the β blocker is one or more selected from the group consisting of carteolol, timolol, nipradilol, a salt of carteolol, a salt of timolol, a salt of nipradilol, a solvate of carteolol or the salt thereof, a solvate of timolol or the salt thereof, and a solvate of nipradilol or the salt thereof.
[5] The aqueous composition according to any of [1] to [4], being an ophthalmic agent.
[6] The aqueous composition according to [5], being an eye drop.
[7] The aqueous composition according to any of [1] to [6], being a prophylactic and/or therapeutic agent for a disease selected from the group consisting of ocular hypertension, glaucoma, and ocular fundus diseases.
[8] The aqueous composition according to any of [1] to [7], further containing one or more selected from the group consisting of α1 receptor blockers, α2 receptor agonists, carbonic anhydrase inhibitors, prostaglandins, sympathomimetics, parasympathomimetics, calcium antagonists, and cholinesterase inhibitors.
[9] The aqueous composition according to any of [1] to [7], further containing one or more selected from the group consisting of latanoprost, dorzolamide, brinzolamide, a salt of latanoprost, a salt of dorzolamide, a salt of brinzolamide, a solvate of latanoprost or the salt thereof, a solvate of dorzolamide or the salt thereof, and a solvate of brinzolamide or the salt thereof.
[10] A pharmaceutical preparation obtained by storing the aqueous composition according to any of [1] to [9] in a container made of polyolefin-based resin.
[11] The pharmaceutical preparation according to [10], wherein the polyolefin-based resin is polyethylene or polypropylene.
[12] The pharmaceutical preparation according to [10] or [11], wherein the container made of polyolefin-based resin is a container for eye drops.
[13] A method for reducing discoloration of an aqueous composition, the method comprising the step of incorporating a β blocker in an aqueous composition comprising a compound represented by Formula (1) or a salt thereof, or a solvate of the compound or the salt thereof.
[14] The method according to [13], wherein the compound represented by Formula (1) is ripasudil.
[15] The method according to [13] or [14], wherein the β blocker is one or more selected from the group consisting of carteolol, timolol, nipradilol, betaxolol, levobunolol, befunolol, metipranolol, a salt of carteolol, a salt of timolol, a salt of nipradilol, a salt of betaxolol, a salt of levobunolol, a salt of befunolol, a salt of metipranolol, a solvate of carteolol or the salt thereof, a solvate of timolol or the salt thereof, a solvate of nipradilol or the salt thereof, a solvate of betaxolol or the salt thereof, a solvate of levobunolol or the salt thereof, a solvate of befunolol or the salt thereof, and a solvate of metipranolol or the salt thereof.
[16] The method according to [13] or [14], wherein the β blocker is one or more selected from the group consisting of carteolol, timolol, nipradilol, a salt of carteolol, a salt of timolol, a salt of nipradilol, a solvate of carteolol or the salt thereof, a solvate of timolol or the salt thereof, and a solvate of nipradilol or the salt thereof.
[17] The method according to any of [13] to [16], wherein the aqueous composition is an ophthalmic agent.
[18] The method according to [17], wherein the ophthalmic agent is an eye drop.
[19] The method according to any of [13] to [18], wherein the aqueous composition is a prophylactic and/or therapeutic agent for a disease selected from the group consisting of ocular hypertension, glaucoma, and ocular fundus diseases.
[20] The method according to any of [13] to [19], wherein the aqueous composition further contains one or more selected from the group consisting of α1 receptor blockers, α2 receptor agonists, carbonic anhydrase inhibitors, prostaglandins, sympathomimetics, parasympathomimetics, calcium antagonists, and cholinesterase inhibitors.
[21] The method according to any of [13] to [19], wherein the aqueous composition further contains one or more selected from the group consisting of latanoprost, dorzolamide, brinzolamide, a salt of latanoprost, a salt of dorzolamide, a salt of brinzolamide, a solvate of latanoprost or the salt thereof, a solvate of dorzolamide or the salt thereof, and a solvate of brinzolamide or the salt thereof.
[22] The method according to any of [13] to [21], further comprising the step of storing the aqueous composition in a container made of polyolefin-based resin.
[23] The method according to [22], wherein the polyolefin-based resin is polyethylene or polypropylene.
[24] The method according to [22] or [23], wherein the container made of polyolefin-based resin is a container for eye drops.
[25] A method for suppressing crystal precipitation of an aqueous composition, the method comprising the step of incorporating a β blocker in an aqueous composition containing a compound represented by Formula (1) or a salt thereof, or a solvate of the compound or the salt thereof.
[26] The method according to [25], wherein the compound represented by Formula (1) is ripasudil.
[27] The method according to [25] or [26], wherein the β blocker is one or more selected from the group consisting of carteolol, timolol, nipradilol, betaxolol, levobunolol, befunolol, metipranolol, a salt of carteolol, a salt of timolol, a salt of nipradilol, a salt of betaxolol, a salt of levobunolol, a salt of befunolol, a salt of metipranolol, a solvate of carteolol or the salt thereof, a solvate of timolol or the salt thereof, a solvate of nipradilol or the salt thereof, a solvate of betaxolol or the salt thereof, a solvate of levobunolol or the salt thereof, a solvate of befunolol or the salt thereof, and a solvate of metipranolol or the salt thereof.
[28] The method according to [25] or [26], wherein the β blocker is one or more selected from the group consisting of carteolol, timolol, nipradilol, a salt of carteolol, a salt of timolol, a salt of nipradilol, a solvate of carteolol or the salt thereof, a solvate of timolol or the salt thereof, and a solvate of nipradilol or the salt thereof.
[29] The method according to any of [25] to [28], wherein the aqueous composition is an ophthalmic agent.
[30] The method according to [29], wherein the ophthalmic agent is an eye drop.
[31] The method according to any of [25] to [30], wherein the aqueous composition is a prophylactic and/or therapeutic agent for a disease selected from the group consisting of ocular hypertension, glaucoma, and ocular fundus diseases.
[32] The method according to any of [25] to [31], wherein the aqueous composition further contains one or more selected from the group consisting of α1 receptor blockers, α2 receptor agonists, carbonic anhydrase inhibitors, prostaglandins, sympathomimetics, parasympathomimetics, calcium antagonists, and cholinesterase inhibitors.
[33] The method according to any of [25] to [31], wherein the aqueous composition further contains one or more selected from the group consisting of latanoprost, dorzolamide, brinzolamide, a salt of latanoprost, a salt of dorzolamide, a salt of brinzolamide, a solvate of latanoprost or the salt thereof, a solvate of dorzolamide or the salt thereof, and a solvate of brinzolamide or the salt thereof.

Further, the present specification discloses, independently from the above invention, the embodiments of invention below, [1A] to [11A], by way of example, from the viewpoint of suppressing the crystal precipitation of an aqueous composition containing a halogenated isoquinoline derivative during low-temperature preservation.

Specifically, to produce a preparation of a halogenated isoquinoline derivative, ripasudil, as an ophthalmic agent or the like, the present inventors initially prepared an aqueous composition containing ripasudil and the preservation stability was investigated, and as a result the aqueous composition was revealed to suffer from crystal precipitation during low-temperature preservation. Thus, the present inventors conducted extensive research to solve the problem, and found that further incorporation of timolol or a salt thereof or a solvate of timolol or the salt thereof in an aqueous composition containing a halogenated isoquinoline derivative such as ripasudil can suppress the crystal precipitation during low-temperature preservation, and that further incorporation of an amino compound can suppress the crystal precipitation during low-temperature preservation over a long period.
[1A] An aqueous composition containing a compound represented by Formula (1): 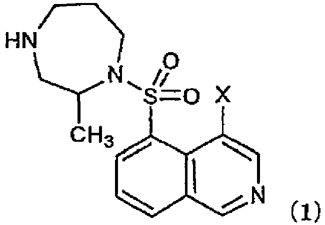 wherein X represents a halogen atom,
   or a salt thereof, or a solvate of the compound or the salt thereof, and timolol or a salt thereof, or a solvate of timolol or the salt thereof.
[2A] The aqueous composition according to [1A], wherein the compound represented by Formula (1) is ripasudil.
[3A] The aqueous composition according to [1A] or [2A], wherein the compound represented by Formula (1) or a salt thereof or a solvate of the compound or the salt thereof is ripasudil hydrochloride hydrate (ripasudil monohydrochloride dihydrate).
[4A] The aqueous composition according to any of [1A] to [3A], wherein the timolol or a salt thereof or a solvate of timolol or the salt thereof is timolol or timolol maleate.
[5A] The aqueous composition according to any of [1A] to [4A], further containing an amino compound.
[6A] The aqueous composition according to [5A], wherein the amino compound is one or more selected from the group consisting of aspartic acid, sodium aspartate, ethyl aminobenzoate, alanine, arginine, arginine hydrochloride, epsilon-aminocaproic acid, ethylenediamine, glycine, glucosamine hydrochloride, glutamine, glutamic acid, glutamic acid hydrochloride, potassium glutamate, sodium glutamate, cystine, cysteine, cysteine hydrochloride, taurine, trometamol, urea, histidine, histidine hydrochloride, phenylalanine, phenprobamate, methionine, monoethanolamine, lysine, lysine hydrochloride, and leucine.
[7A] The aqueous composition according to any of [1A] to [6A], wherein the content of the compound represented by Formula (1) is 0.01 to 10 w/v%, calculated as the free form of the compound.
[8A] The aqueous composition according to any of [1A] to [7A], wherein the content of the timolol or a salt thereof or a solvate of timolol or the salt thereof is 0.01 to 5 w/v%, calculated as the free form of timolol.
[9A] The aqueous composition according to any of [1A] to [8A], further containing one or more selected from the group consisting of α1 receptor blockers, α2 receptor agonists, β blockers, carbonic anhydrase inhibitors, prostaglandins, sympathomimetics, parasympathomimetics, calcium antagonists, and cholinesterase inhibitors.
[10A] The aqueous composition according to any of [1A] to [9A], further containing one or more selected from the group consisting of tafluprost, travoprost, bimatoprost, latanoprost, nipradilol, bunazosin, brimonidine, dorzolamide, brinzolamide, isopropyl unoprostone, a salt of tafluprost, a salt of travoprost, a salt of bimatoprost, a salt of latanoprost, a salt of nipradilol, a salt of bunazosin, a salt of brimonidine, a salt of dorzolamide, a salt of brinzolamide, a salt of isopropyl unoprostone, a solvate of tafluprost or the salt thereof, a solvate of travoprost or the salt thereof, a solvate of bimatoprost or the salt thereof, a solvate of latanoprost or the salt thereof, a solvate of nipradilol or the salt thereof, a solvate of bunazosin or the salt thereof, a solvate of brimonidine or the salt thereof, a solvate of dorzolamide or the salt thereof, a solvate of brinzolamide or the salt thereof, and a solvate of isopropyl unoprostone or the salt thereof.
[11A] A crystal precipitation-suppressing agent for an aqueous composition containing a compound represented by Formula (1) or a salt thereof, or a solvate of the compound or the salt thereof, and timolol or a salt thereof, or a solvate of timolol or the salt thereof.

The embodiments of invention, [1A] to [11A], can advantageously suppress the crystal precipitation of an aqueous composition containing a halogenated isoquinoline derivative during low-temperature preservation.

Furthermore, the present specification discloses, independently from the above invention, the embodiments of invention below, [1B] to [31B], by way of example, from the viewpoint of suppressing the decomposition of timolol in an aqueous composition containing a halogenated isoquinoline derivative and timolol.

Specifically, the present inventors intended to develop a combination drug of timolol and a halogenated isoquinoline derivative, and conducted extensive research to establish a technique to produce such a preparation, and as a result it was revealed that, when an aqueous composition containing both of the components was preserved at high temperature over a long period, timolol decomposed to form a degradation product. Thus, the present inventors conducted extensive research to solve the problem, and found that the stability of timolol is influenced by the pH value, and a pH value of 7.0, typically set for an aqueous composition containing timolol singly, or a pH value slightly higher than the pH value allows formation of a degradation product of timolol, and in contrast lowering the pH value of an aqueous composition to 6.5 or lower suppresses the decomposition of timolol.
[1B] An aqueous composition containing:
   Component (A) as a compound represented by Formula (1): 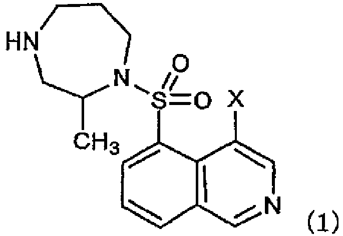 wherein X represents a halogen atom,
      or a salt thereof, or a solvate of the compound or the salt thereof; and
      Component (B) as timolol or a salt thereof, or a solvate of timolol or the salt thereof;
      and wherein the pH value of the aqueous composition is 6.5 or lower (4.0 to 6.5, for example).
[2B] The aqueous composition according to [1B], wherein the compound represented by Formula (1) is ripasudil.
[3B] The aqueous composition according to [1B], wherein the pH value is 6.0 or lower (4.0 to 6.0, for example).
[4B] The aqueous composition according to [1B], wherein the pH value is 5.5 or lower (4.0 to 5.5, for example).
[5B] The aqueous composition according to [1B], wherein the pH value is 5.0 or lower (4.0 to 5.0, for example).
[6B] The aqueous composition according to [1B], wherein the pH value is 4.5 or lower (4.0 to 4.5, for example).
[7B] The aqueous composition according to any of [1B] to [6B], being an ophthalmic agent.
[8B] The aqueous composition according to [7B], being an eye drop.
[9B] The aqueous composition according to any of [1B] to [8B], being a prophylactic and/or therapeutic agent for a disease selected from the group consisting of ocular hypertension and glaucoma.
[10B] The aqueous composition according to any of [1B] to [9B], further containing one or more selected from the group consisting of α1 receptor blockers, α2 receptor agonists, β blockers, carbonic anhydrase inhibitors, prostaglandins, sympathomimetics, parasympathomimetics, calcium antagonists, and cholinesterase inhibitors.
[11B] The aqueous composition according to any of [1B] to [9B], further containing one or more selected from the group consisting of latanoprost, nipradilol, dorzolamide, brinzolamide, a salt of latanoprost, a salt of nipradilol, a salt of dorzolamide, a salt of brinzolamide, a solvate of latanoprost of the salt thereof, a solvate of nipradilol or the salt thereof, a solvate of dorzolamide or the salt thereof, and a solvate of brinzolamide or the salt thereof.
[12B] A method for stabilizing (preferably, a method for suppressing decomposition of) timolol or a salt thereof or a solvate of timolol or the salt thereof in an aqueous composition, the method comprising the step of adjusting the pH value of an aqueous composition to 6.5 or lower (4.0 to 6.5, for example), wherein the aqueous composition containing:
   Component (A) as a compound represented by Formula (1) or a salt thereof, or a solvate of the compound or the salt thereof; and
   Component (B) as timolol or a salt thereof, or a solvate of timolol or the salt thereof.
[13B] The method according to [12B], wherein the compound represented by Formula (1) is ripasudil.
[14B] The method according to [12B], wherein the pH value is 6.0 or lower (4.0 to 6.0, for example).
[15B] The method according to [12B], wherein the pH value is 5.5 or lower (4.0 to 5.5, for example).
[16B] The method according to [12B], wherein the pH value is 5.0 or lower (4.0 to 5.0, for example).
[17B] The method according to [12B], wherein the pH value is 4.5 or lower (4.0 to 4.5, for example).
[18B] The method according to any of [12B] to [17B], wherein the aqueous composition is an ophthalmic agent.
[19B] The method according to [18B], wherein the ophthalmic agent is an eye drop.
[20B] The method according to any of [12B] to [19B], wherein the aqueous composition is a prophylactic and/or therapeutic agent for a disease selected from the group consisting of ocular hypertension and glaucoma.
[21B] The method according to any of [12B] to [20B], wherein the aqueous composition further contains one or more selected from the group consisting of α1 receptor blockers, α2 receptor agonists, β blockers, carbonic anhydrase inhibitors, prostaglandins, sympathomimetics, parasympathomimetics, calcium antagonists, and cholinesterase inhibitors.
[22B] The method according to any of [12B] to [20B], wherein the aqueous composition further contains one or more selected from the group consisting of latanoprost, nipradilol, dorzolamide, brinzolamide, a salt of latanoprost, a salt of nipradilol, a salt of dorzolamide, a salt of brinzolamide, a solvate of latanoprost or the salt thereof, a solvate of nipradilol or the salt thereof, a solvate of dorzolamide or the salt thereof, and a solvate of brinzolamide or the salt thereof.
[23B] A method for stabilizing (preferably, a method for suppressing decomposition of) a compound represented by Formula (1) or a salt thereof or a solvate of the compound or the salt thereof in an aqueous composition, the method comprising the step of adjusting the pH value of an aqueous composition to 5.5 or lower (4.0 to 5.5, for example), wherein the aqueous composition containing:
   Component (A) as a compound represented by Formula (1) or a salt thereof, or a solvate of the compound or the salt thereof; and
   Component (B) as timolol or a salt thereof, or a solvate of timolol or the salt thereof.
[24B] The method according to [23B], wherein the compound represented by Formula (1) is ripasudil.
[25B] The method according to [23B], wherein the pH value is 5.0 or lower (4.0 to 5.0, for example).
[26B] The method according to [23B], wherein the pH value is 4.5 or lower (4.0 to 4.5, for example).
[27B] The method according to any of [23B] to [26B], wherein the aqueous composition is an ophthalmic agent.
[28B] The method according to [27B], wherein the ophthalmic agent is an eye drop.
[29B] The method according to any of [23B] to [28B], wherein the aqueous composition is a prophylactic and/or therapeutic agent for a disease selected from the group consisting of ocular hypertension and glaucoma.
[30B] The method according to any of [23B] to [29B], wherein the aqueous composition further contains one or more selected from the group consisting of α1 receptor blockers, α2 receptor agonists, β blockers, carbonic anhydrase inhibitors, prostaglandins, sympathomimetics, parasympathomimetics, calcium antagonists, and cholinesterase inhibitors.
[31B] The method according to any of [23B] to [29B], wherein the aqueous composition further contains one or more selected from the group consisting of latanoprost, nipradilol, dorzolamide, brinzolamide, a salt of latanoprost, a salt of nipradilol, a salt of dorzolamide, a salt of brinzolamide, a solvate of latanoprost or the salt thereof, a solvate of nipradilol or the salt thereof, a solvate of dorzolamide or the salt thereof, and a solvate of brinzolamide or the salt thereof.

The embodiments of invention, [1B] to [31B], can advantageously suppress the decomposition of the components of an aqueous composition containing a halogenated isoquinoline derivative and timolol.

### <Compound represented by Formula (1)>

Examples of the halogen atom in Formula (1) include a fluorine atom, a chlorine atom, and a bromine atom. In Formula (1), a fluorine atom or a bromine atom is preferred as the halogen atom, and a fluorine atom is particularly preferred.

Further, in Formula (1), the carbon atom forming the homopiperazine ring substituted with the methyl group is an asymmetric carbon atom. As a result, stereoisomerism occurs. The compound represented by Formula (1) includes all the stereoisomers, and may be a single stereoisomer or a mixture of various stereoisomers at any given ratio. Preferred as the compound represented by Formula (1) is a compound having the S configuration as the absolute configuration.

The salt of the compound represented by Formula (1) is not particularly limited as long as it is a pharmacologically acceptable salt, and specific examples of the salt include inorganic acid salts such as hydrochloride, sulfate, nitrate, hydrofluoride, and hydrobromate; and organic acid salts such as acetate, tartrate, lactate, citrate, fumarate, maleate, succinate, methanesulfonate, ethanesulfonate, benzenesulfonate, toluenesulfonate, naphthalenesulfonate, and camphorsulfonate, with hydrochloride being preferred.

The compound represented by Formula (1) or a salt thereof may also be in the form of a hydrate or a solvate such as an alcohol solvate, and is preferably in the form of a hydrate.

Specific examples of the compound represented by Formula (1) or a salt thereof or a solvate of the compound or the salt thereof include:
ripasudil (chemical name: 4-fluoro-5-{[(2S)-2-methyl-1,4-diazepan-1-yl]sulfonyl}isoquinoline) or a salt thereof or a solvate of ripasudil or the salt thereof; and
4-bromo-5-{[(2S)-2-methyl-1,4-diazepan-1-yl]sulfonyl}isoquinoline or a salt thereof or a solvate of 4-bromo-5-{[(2S)-2-methyl-1,4-diazepan-1-yl]sulfonyl}isoquinoline or the salt thereof.

The compound represented by Formula (1) or a salt thereof or a solvate of the compound or the salt thereof is preferably ripasudil or a salt thereof or a solvate of ripasudil or the salt thereof, or 4-bromo-5-{[(2S)-2-methyl-1,4-diazepan-1-yl]sulfonyl}isoquinoline or a salt thereof or a solvate of 4-bromo-5-{[(2S)-2-methyl-1,4-diazepan-1-yl]sulfonyl]isoquinoline or the salt thereof, more preferably ripasudil or a salt thereof or a solvate of ripasudil or the salt thereof, still more preferably ripasudil or a hydrochloride thereof or a hydrate of ripasudil or the hydrochloride thereof, and particularly preferably a ripasudil hydrochloride hydrate (ripasudil monohydrochloride dihydrate) represented by the following structural formula:

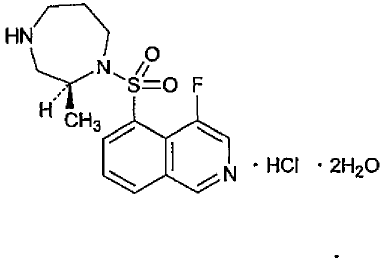

The compound represented by Formula (1) or a salt thereof or a solvate of the compound or the salt thereof is known and can be produced using a known method. Specifically, ripasudil or a salt thereof or a solvate of ripasudil or the salt thereof can be produced using the method described in WO1999/020620 or WO2006/057397, for example. 4-Bromo-5-{[(2S)-2-methyl-1,4-diazepan-1-yl]sulfonyl}isoquinoline or a salt thereof or a solvate of 4-bromo-5-{[(2S)-2-methyl-1,4-diazepan-1-yl]sulfonyl}isoquinoline or the salt thereof can be produced using the method described in WO2006/115244, for example.

The content of the compound represented by Formula (1) or a salt thereof or a solvate of the compound or the salt thereof in the aqueous composition is not particularly limited, and may be determined as appropriate, in consideration of the target disease, or the sex, age, or symptoms of the patient, for example. From the viewpoint of achieving excellent pharmacological action, however, the content of the compound represented by Formula (1) or a salt thereof or a solvate of the compound or the salt thereof is preferably 0.01 to 10 w/v%, more preferably 0.02 to 8 w/v%, and particularly preferably 0.04 to 6 w/v%, calculated as the free form of the compound represented by Formula (1), based on the total volume of the aqueous composition. In particular, when ripasudil is used as the compound represented by Formula (1), from the viewpoint of achieving excellent pharmacological action, the content of ripasudil or a salt thereof or a solvate of ripasudil or the salt thereof is preferably 0.05 to 5 w/v%, more preferably 0.1 to 3 w/v%, and particularly preferably 0.15 to 2 w/v%, calculated as the free form, based on the total volume of the aqueous composition.

### <β blocker>

As used herein, the "β blocker" is not particularly limited as long as it has β-blocking action, and specific examples of such β blockers include carteolol, timolol, nipradilol, betaxolol, levobunolol, befunolol, metipranolol, pharmaceutically acceptable salts thereof, and solvates of such a compound or pharmaceutically acceptable salt thereof with water, an alcohol, or the like, and these can be used singly or in combination of two or more. The pharmaceutically acceptable salt is not particularly limited, and specific examples of the pharmaceutically acceptable salt include inorganic acid salts such as hydrochloride, sulfate, nitrate, hydrofluoride, and hydrobromate; and organic acid salts such as acetate, tartrate, lactate, citrate, fumarate, maleate, succinate, methanesulfonate, ethanesulfonate, benzenesulfonate, toluenesulfonate, naphthalenesulfonate, and camphorsulfonate.

Preferred as the β blocker is one or more selected from the group consisting of carteolol, timolol, nipradilol, a salt of carteolol, a salt of timolol, a salt of nipradilol, a solvate of carteolol or the salt thereof, a solvate of timolol or the salt thereof, and a solvate of nipradilol or the salt thereof, more preferred is one or more selected from the group consisting of carteolol hydrochloride (chemical name: 5-[(2RS)-3-(1,1-dimethylethyl)amino-2-hydroxypropyloxy]-3,4-dihydroquinolin-2(1H)-one monohydrochloride), timolol maleate (chemical name: (2S)-1-[(1,1-dimethylethyl)amino]-3-(4-morpholin-4-yl-1,2,5-thiadiazol-3-yloxy)propan-2-ol monomaleate), and nipradilol (chemical name: 3,4-dihydro-8-(2-hydroxy-3-isopropylamino)propoxy-3-nitroxy-2H-1-benzopyran), and particularly preferred is one or more selected from the group consisting of carteolol hydrochloride and nipradilol.

Each of these β blockers is known, and may be produced using a known method, or a commercially available product may be used.

The β blocker has reducing action for discoloration during high-temperature preservation, and in addition suppressing action for crystal precipitation during low-temperature preservation. As specifically disclosed in Test Examples 2, 3, and 4, it was found that, although the aqueous composition containing the compound represented by Formula (1) typified by ripasudil or a salt thereof or a solvate of ripasudil or the salt thereof can suffer from crystal precipitation during low-temperature preservation, further incorporation of carteolol, timolol, or nipradilol in the aqueous composition suppresses crystal precipitation during low-temperature preservation in comparison with the case where the aqueous composition does not contain any of them. Accordingly, the aqueous composition containing the compound represented by Formula (1) or a salt thereof or a solvate of the compound or the salt thereof and the β blocker undergoes reduced discoloration during high-temperature preservation, and crystal precipitation during low-temperature preservation is also suppressed, and thus has an advantage of excellent preservation stability.

While the content of the β blocker in the aqueous composition is not particularly limited, from the viewpoint of discoloration-reducing action and/or crystal precipitation-suppressing action, the content of the P blocker is preferably 0.01 to 10 w/v%, more preferably 0.04 to 4 w/v%, and particularly preferably 0.075 to 2.5 w/v% based on the total volume of the aqueous composition.

While the content ratio by mass of the β blocker to the compound represented by Formula (1) or a salt thereof or a solvate of the compound or the salt thereof in the aqueous composition is not particularly limited, from the viewpoint of discoloration-reducing action and/or crystal precipitation-suppressing action, the content ratio by mass of the β blocker is preferably 0.02 to 30 parts by mass, more preferably 0.2 to 15 parts by mass, and particularly preferably 0.5 to 10 parts by mass, with respect to 1 part by mass of the compound represented by Formula (1) calculated as the free form. In particular, when the compound represented by Formula (1) or a salt thereof or a solvate of the compound or the salt thereof is ripasudil or a salt thereof or a solvate of ripasudil or the salt thereof, from the viewpoint of discoloration-reducing action and/or crystal precipitation-suppressing action, the content ratio by mass of the β blocker is preferably 0.005 to 20 parts by mass, more preferably 0.005 to 12.5 parts by mass, and particularly preferably 0.25 to 7.5 parts by mass, with respect to 1 part by mass of ripasudil or a salt thereof or a solvate of ripasudil or the salt thereof, calculated as the free form.

In particular, when the β blocker is carteolol or a salt thereof or a solvate of carteolol or the salt thereof, from the viewpoint of discoloration-reducing action and/or crystal precipitation-suppressing action, the content of carteolol or a salt thereof or a solvate of carteolol or the salt thereof is preferably 0.1 to 5 w/v%, more preferably 0.5 to 3 w/v%, and particularly preferably 1 to 2 w/v%, calculated as the free form of carteolol, based on the total volume of the aqueous composition.

While the content ratio by mass of carteolol or a salt thereof or a solvate of carteolol or the salt thereof to the compound represented by Formula (1) or a salt thereof or a solvate of the compound or the salt thereof in the aqueous composition is not particularly limited, from the viewpoint of discoloration-reducing action and/or crystal precipitation-suppressing action, the content ratio by mass of carteolol or a salt thereof or a solvate of carteolol or the salt thereof is preferably 0.25 to 20 parts by mass, more preferably 0.75 to 12.5 parts by mass, and particularly preferably 1.5 to 7.5 parts by mass, calculated as the free form, with respect to 1 part by mass of the compound represented by Formula (1) calculated as the free form. In particular, when the compound represented by Formula (1) or a salt thereof or a solvate of the compound or the salt thereof is ripasudil or a salt thereof or a solvate of ripasudil or the salt thereof, from the viewpoint of discoloration-reducing action and/or crystal precipitation-suppressing action, the content ratio by mass of carteolol or a salt thereof or a solvate of carteolol or the salt thereof is preferably 0.5 to 15 parts by mass, more preferably 1 to 10 parts by mass, and particularly preferably 2 to 5 parts by mass, calculated as the free form, with respect to 1 part by mass of ripasudil or a salt thereof or a solvate of ripasudil or the salt thereof, calculated as the free form.

When the β blocker is timolol or a salt thereof or a solvate thereof, from the viewpoint of discoloration-reducing action and/or crystal precipitation-suppressing action, the content of timolol or a salt thereof or a solvate of timolol or the salt thereof is preferably 0.01 to 5 w/v%, more preferably 0.02 to 2 w/v%, preferably 0.05 to 0.75 w/v%, and particularly preferably 0.25 to 0.5 w/v%, calculated as the free form of timolol, based on the total volume of the aqueous composition.

While the content ratio by mass of timolol or a salt thereof or a solvate of timolol or the salt thereof to the compound represented by Formula (1) or a salt thereof or a solvate of the compound or the salt thereof in the aqueous composition is not particularly limited, from the viewpoint of discoloration-reducing action and/or crystal precipitation-suppressing action, the content ratio by mass of timolol or a salt thereof or a solvate of timolol or the salt thereof is preferably 0.05 to 6 parts by mass, more preferably 0.3 to 4 parts by mass, and particularly preferably 0.6 to 2 parts by mass, calculated as the free form, with respect to 1 part by mass of the compound represented by Formula (1), calculated as the free form. In particular, when the compound represented by Formula (1) or a salt thereof or a solvate of the compound or the salt thereof is ripasudil or a salt thereof or a solvate of ripasudil or the salt thereof, from the viewpoint of discoloration-reducing action and/or crystal precipitation-suppressing action, the content ratio by mass of timolol or a salt thereof or a solvate of timolol or the salt thereof is preferably 0.01 to 5 parts by mass, more preferably 0.1 to 3 parts by mass, and particularly preferably 0.5 to 1.5 parts by mass, calculated as the free form, with respect to 1 part by mass of ripasudil or a salt thereof or a solvate of ripasudil or the salt thereof, calculated as the free form.

Further, when the β blocker is nipradilol or a salt thereof or a solvate of nipradilol or the salt thereof, from the viewpoint of discoloration-reducing action and/or crystal precipitation-suppressing action, the content of nipradilol or a salt thereof or a solvate of nipradilol or the salt thereof is preferably 0.05 to 2 w/v%, more preferably 0.1 to 1 w/v%, and particularly preferably 0.3 to 0.5 w/v%, calculated as the free form of nipradilol, based on the total volume of the aqueous composition.

While the content ratio by mass of nipradilol or a salt thereof or a solvate of nipradilol or the salt thereof to the compound represented by Formula (1) or a salt thereof or a solvate of the compound or the salt thereof in the aqueous composition is not particularly limited, from the viewpoint of discoloration-reducing action and/or crystal precipitation-suppressing action, the content ratio by mass of nipradilol or a salt thereof or a solvate of nipradilol or the salt thereof is preferably 0.025 to 5 parts by mass, more preferably 0.075 to 2 parts by mass, and particularly preferably 0.25 to 1 parts by mass, calculated as the free form of nipradilol or a salt thereof or a solvate of nipradilol or the salt thereof, with respect to 1 part by mass of the compound represented by Formula (1) calculated as the free form. In particular, when the compound represented by Formula (1) or a salt thereof or a solvate of the compound or the salt thereof is ripasudil or a salt thereof or a solvate of ripasudil or the salt thereof, from the viewpoint of discoloration-reducing action and/or crystal precipitation-suppressing action, the content ratio by mass of nipradilol or a salt thereof or a solvate of nipradilol or the salt thereof is preferably 0.05 to 3 parts by mass, more preferably 0.1 to 1.5 parts by mass, and particularly preferably 0.5 to 0.75 parts by mass, calculated as the free form, with respect to 1 part by mass of ripasudil or a salt thereof or a solvate of ripasudil or the salt thereof, calculated as the free form.

### <Amino compound>

It is preferred to further incorporate an amino compound in the aqueous composition (in particular, the aqueous composition containing timolol or a salt thereof or a solvate of timolol or the salt thereof as a β blocker). As disclosed in Test Example 5 below, incorporation of an amino compound was found to provide the aqueous composition with excellent crystal precipitation-suppressing effect when the aqueous composition is preserved at low temperature over a long period.

The "amino compound" is a compound having an amino group or a salt thereof, and not particularly limited herein as long as it has been confirmed to be basically safe for administration to a human body, and specific examples of such amino compounds include aspartic acid, sodium aspartate, ethyl aminobenzoate, alanine, arginine, arginine hydrochloride, epsilon-aminocaproic acid, ethylenediamine, glycine, glucosamine hydrochloride, glutamine, glutamic acid, glutamic acid hydrochloride, potassium glutamate, sodium glutamate, cystine, cysteine, cysteine hydrochloride, taurine, trometamol, urea, histidine, histidine hydrochloride, phenylalanine, phenprobamate, methionine, monoethanolamine, lysine, lysine hydrochloride, and leucine, and these can be used singly or in combination of two or more. Each of them is a known compound, and can be produced using a known method, or a commercially available product can be used.

Preferred as the amino compound is epsilon-aminocaproic acid, glutamic acid, sodium glutamate, taurine, trometamol, or monoethanolamine.

The content of the amino compound in the aqueous composition is not particularly limited, and may be determined on the basis of the contents of the compound represented by Formula (1) or a salt thereof or a solvate of the compound or the salt thereof and the β blocker (in particular, timolol or a salt thereof or a solvate of timolol or the salt thereof) in the aqueous composition determined as appropriate, in consideration of the target disease, or the sex, age, or symptoms of the patient, for example, and crystal precipitation-suppressing effect intended. From the viewpoint of achieving excellent crystal precipitation-suppressing action, the content of the amino compound is preferably 0.01 to 20 w/v%, more preferably 0.2 to 10 w/v%, and particularly preferably 0.25 to 1.5 w/v% based on the total volume of the aqueous composition. When monoethanolamine is used as the amino compound, the content of monoethanolamine is preferably 0.05 to 5 w/v%, more preferably 0.1 to 2 w/v%, and particularly preferably 0.5 to 1 w/v% based on the total volume of the aqueous composition.

### <Aqueous composition>

As used herein, the "aqueous composition" means a composition containing at least water, which may be in the form of a liquid (solution or suspension) or a semisolid (ointment), for example, and preferably in the form of a liquid. As the water in the composition, purified water, water for injection, or sterile purified water, for example, can be used.

While the content of water in the aqueous composition is not particularly limited, it is preferably 5 mass% or more, more preferably 20 mass% or more, still more preferably 50 mass% or more, even more preferably 90 mass% or more, and particularly preferably 90 to 99.8 mass%.

The aqueous composition can be prepared into various dosage forms in accordance with known methods described in the General Rules for Preparations in the Japanese Pharmacopoeia 16^{th} Edition, for example. Examples of dosage forms include injections, inhalation liquids, eye drops, eye ointments, ear drops, nasal drops, enemas, liquids for external use, sprays, ointments, gels, oral liquids, and syrups. From the viewpoint of advantageously utilizing the pharmacological action of the compound represented by Formula (1), the dosage form is an ophthalmic agent, which specifically is preferably an eye drop or an eye ointment, and is particularly preferably an eye drop.

The aqueous composition may contain, in addition to the components described above, additives used in drugs, quasi drugs, and the like, in accordance with the dosage form. Examples of such additives include inorganic salts, isotonic agents, chelating agents, stabilizers, pH regulators, antiseptics, antioxidants, thickeners, surfactants, solubilizers, suspending agents, cooling agents, dispersants, preservatives, oily bases, emulsion bases, and water-soluble bases. For such an additive, a component corresponding to the above "amino compound" may be used.

Specific examples of these additives include ascorbic acid, potassium aspartate, sodium bisulfite, alginic acid, sodium benzoate, benzyl benzoate, epsilon-aminocaproic acid, fennel oil, ethanol, ethylene-vinyl acetate copolymer, sodium edetate, tetrasodium edetate, potassium chloride, calcium chloride hydrate, sodium chloride, magnesium chloride, hydrochloric acid, alkyl diaminoethylglycine hydrochloride solution, carboxyvinyl polymer, dried sodium sulfite, dried sodium carbonate, d-camphor, dl-camphor, xylitol, citric acid hydrate, sodium citrate hydrate, glycerin, gluconic acid, L-glutamic acid, monosodium L-glutamate, creatinine, chlorhexidine gluconate solution, chlorobutanol, sodium dihydrogenphosphate dihydrate, geraniol, sodium chondroitin sulfate, acetic acid, potassium acetate, sodium acetate hydrate, titanium oxide, gellan gum, dibutylhydroxytoluene, potassium bromide, benzododecinium bromide, tartaric acid, sodium hydroxide, polyoxyl 45 stearate, purified lanolin, D-sorbitol, sorbitol solution, taurine, sodium bicarbonate, sodium carbonate hydrate, sodium thiosulfate hydrate, thimerosal, tyloxapol, sodium dehydroacetate, trometamol, concentrated glycerin, mixed tocopherol concentrate, white petrolatum, mentha water, mentha oil, benzalkonium chloride concentrated solution 50, ethyl parahydroxybenzoate, butyl parahydroxybenzoate, propyl parahydroxybenzoate, methyl parahydroxybenzoate, sodium hyaluronate, human serum albumin, hydroxyethyl cellulose, hydroxypropyl cellulose, hypromellose, glacial acetic acid, sodium pyrosulfite, phenylethyl alcohol, glucose, propylene glycol, bergamot oil, benzalkonium chloride, benzalkonium chloride solution, benzyl alcohol, benzethonium chloride, benzethonium chloride solution, borax, boric acid, povidone, polyoxyethylene (200) polyoxypropylene glycol (70), sodium polystyrene sulfonate, polysorbate 80, polyoxyethylene hydrogenated castor oil 60, partially hydrolyzed polyvinyl alcohol, d-borneol, macrogol 4000, macrogol 6000, D-mannitol, anhydrous citric acid, anhydrous sodium monohydrogen phosphate, anhydrous sodium dihydrogen phosphate, methanesulfonic acid, methylcellulose, 1-menthol, monoethanolamine, aluminum monostearate, polyethylene glycol monostearate, eucalyptus oil, potassium iodide, sulfuric acid, oxyquinoline sulfate, liquid paraffin, borneo camphor, phosphoric acid, dibasic sodium phosphate hydrate, potassium dihydrogenphosphate, sodium dihydrogenphosphate, sodium dihydrogenphosphate monohydrate, malic acid, and petrolatum.

Examples of preferred additives include potassium chloride, calcium chloride hydrate, sodium chloride, magnesium chloride, glycerin, acetic acid, potassium acetate, sodium acetate hydrate, tartaric acid, sodium hydroxide, sodium bicarbonate, sodium carbonate hydrate, concentrated glycerin, hydroxyethyl cellulose, hydroxypropyl cellulose, hypromellose, borax, boric acid, povidone, polysorbate 80, polyoxyethylene hydrogenated castor oil, polyethylene glycol monostearate, partially hydrolyzed polyvinyl alcohol, macrogol 4000, macrogol 6000, anhydrous citric acid, anhydrous sodium monohydrogen phosphate, anhydrous sodium dihydrogen phosphate, methylcellulose, monoethanolamine, phosphoric acid, dibasic sodium phosphate hydrate, potassium dihydrogenphosphate, sodium dihydrogenphosphate, sodium dihydrogenphosphate monohydrate, sodium hyaluronate, glucose, and 1-menthol.

The aqueous composition may further contain, in addition to the components described above, other medicinal components in accordance with the target disease and the like. Examples of such medicinal components include α1 receptor blockers including bunazosin or a salt thereof or a solvate of bunazosin or the salt thereof, such as bunazosin hydrochloride; α2 receptor agonists including brimonidine or a salt thereof or a solvate of brimonidine or the salt thereof, such as brimonidine tartrate, and apraclonidine or a salt thereof or a solvate of apraclonidine or the salt thereof; carbonic anhydrase inhibitors including dorzolamide or a salt thereof or a solvate of dorzolamide or the salt thereof, such as dorzolamide hydrochloride, brinzolamide or a salt thereof or a solvate of brinzolamide or the salt thereof, acetazolamide or a salt thereof or a solvate of acetazolamide or the salt thereof, dichlorphenamide or a salt thereof or a solvate of dichlorphenamide or the salt thereof, and methazolamide or a salt thereof or a solvate of methazolamide or the salt thereof; prostaglandins including isopropyl unoprostone or a salt thereof or a solvate of isopropyl unoprostone or the salt thereof, tafluprost or a salt thereof or a solvate of tafluprost or the salt thereof, travoprost or a salt thereof or a solvate of travoprost or the salt thereof, bimatoprost or a salt thereof or a solvate of bimatoprost or the salt thereof, and latanoprost or a salt thereof or a solvate of latanoprost or the salt thereof; sympathomimetics including dipivefrine or a salt thereof or a solvate of dipivefrine or the salt thereof, such as dipivefrine hydrochloride, and epinephrine or a salt thereof or a solvate of epinephrine or the salt thereof, such as epinephrine, epinephrine borate, or epinephrine hydrochloride; parasympathomimetics including distigmine bromide or a salt thereof or a solvate of distigmine bromide or the salt thereof, pilocarpine or a salt thereof or a solvate of pilocarpine or the salt thereof, such as pilocarpine, pilocarpine hydrochloride, or pilocarpine nitrate, and carbachol or a salt thereof or a solvate of carbachol or the salt thereof; calcium antagonists including lomerizine or a salt thereof or a solvate of lomerizine or the salt thereof, such as lomerizine hydrochloride; and cholinesterase inhibitors including demecarium or a salt thereof or a solvate of demecarium or the salt thereof, echothiophate or a salt thereof or a solvate of echothiophate or the salt thereof, and physostigmine or a salt thereof or a solvate of physostigmine or the salt thereof. One or more of these medicinal components can be incorporated.

Preferred as the other medicinal components is one or more selected from the group consisting of latanoprost, dorzolamide, brinzolamide, a salt of latanoprost, a salt of dorzolamide, a salt of brinzolamide, a solvate of latanoprost or the salt thereof, a solvate of dorzolamide or the salt thereof, and a solvate of brinzolamide or the salt thereof.

The pH of the aqueous composition is not particularly limited, but is preferably 4 to 9, more preferably 4.5 to 8, and particularly preferably 5 to 7. The osmotic pressure ratio of the aqueous composition relative to physiological saline is not particularly limited, but is preferably 0.6 to 3, and particularly preferably 0.6 to 2.

When the aqueous composition contains timolol or a salt thereof or a solvate of timolol or the salt thereof as a β blocker, the pH value is preferably 6.5 or lower.

As disclosed in Test Examples 7 and 8 below, it was found that, while preservation of the aqueous composition containing the compound represented by Formula (1) or a salt thereof or a solvate of the compound or the salt thereof and timolol or a salt thereof or a solvate of timolol or the salt thereof at high temperature over a long period results in decomposition of timolol to form a degradation product, setting the pH value of the aqueous composition to 6.5 or lower suppresses the decomposition of timolol to achieve stabilization, and setting the pH value to a further lower value further stabilizes the compound represented by Formula (1) or a salt thereof or a solvate of the compound or the salt thereof in addition to timolol.

As used herein, the "pH value" means a value obtained by measuring the pH of the aqueous composition at a temperature of 25°C. While the pH value is preferably 6.5 or lower, from the viewpoint of the stability of timolol or a salt thereof or a solvate of timolol or the salt thereof, and in addition the stability of the compound represented by Formula (1) (preferably ripasudil) or a salt thereof or a solvate of the compound or the salt thereof, the pH value is more preferably 6.0 or lower, still more preferably 5.5 or lower, even more preferably 5.0 or lower, and particularly preferably 4.5 or lower.

The means to adjust the pH value of the aqueous composition is not particularly limited, and those skilled in the art could appropriately adjust the pH value by adjusting the type and amount of each component (pH regulator, for example) to be incorporated while checking the pH value.

The aqueous composition is preferably stored in a container, from the viewpoint of preservation stability and portability, and the like. As used herein, the "container" means a package for directly storing the aqueous composition. The container is a concept that includes all of the "well-closed container", "tight container", and "hermetic container" defined in the General Notices in the Japanese Pharmacopoeia 16^{th} Edition.

The form of the container is not particularly limited as long as it can store an aqueous composition, and may be selected or set as appropriate, depending on the dosage form, for example. Specific examples of such containers include containers for injections, containers for inhalations, containers for sprays, bottle-shaped containers, tubular containers, containers for eye drops, containers for nasal drops, containers for ear drops, and bag containers. The container may be further packaged in a box, a bag, or the like.

The material of the container is not particularly limited, and may be selected as appropriate depending on the form of the container. Specific examples of materials include glass, plastics, cellulose, pulp, rubber, and metals, and preferably a plastic from the viewpoint of processability, squeezability, and durability. The resin for a container made of a plastic is preferably a thermoplastic resin. Examples of such resins include polyolefin-based resins such as low-density polyethylene (including linear low-density polyethylene), high-density polyethylene, medium-density polyethylene, polypropylene, and cyclic polyolefins; polyester-based resins such as polyethylene terephthalate, polybutylene terephthalate, polyethylene naphthalate, polybutylene naphthalate, and poly(1,4-cyclohexylene dimethylene terenaphthalate); polyphenylene ether-based resins; polycarbonate-based resins; polysulfone-based resins; polyamide-based resins; polyvinyl chloride resins; and styrene-based resins. A mixture of these resins (polymer alloy) may also be used.

While the material of the container is not particularly limited, from the viewpoint of discoloration-reducing action, it is preferably a polyolefin-based resin, and particularly preferably polypropylene. As described in Test Examples below, discoloration is particularly prominently reduced in the case of a container made of polyolefin-based resin.

As used herein, the "container made of polyolefin-based resin" means a container in which at least a portion that contacts the aqueous composition is "made of polyolefin-based resin". Accordingly, a container in which, for example, a polyolefin layer is provided as the inner layer that contacts the aqueous composition and a different resin material or the like is, for example, laminated on the outer side of the inner layer also corresponds to the "container made of polyolefin-based resin". The polyolefin-based resin is not particularly limited herein, and may be a polymer of a single monomer (homopolymer) or a copolymer of a plurality of monomers (copolymer). In the case of a copolymer, the mode of polymerization is not particularly limited, and may be random polymerization or block polymerization. Further, the stereoregularity (tacticity) of the polyolefin-based resin is not particularly limited.

Specific examples of such polyolefin-based resins include polyethylene (more specifically, such as low-density polyethylene (including linear low density polyethylene), high-density polyethylene, and medium-density polyethylene), polypropylene, cyclic polyolefins, poly(4-methylpentene), polytetrafluoroethylene, ethylenepropylene copolymer, ethylene-α-olefin copolymer, ethylene-acrylic acid copolymer, ethylene-methacrylic acid copolymer, ethylene-vinylacetate copolymer, and ethylene-ethyl acrylate copolymer. These polyolefin-based resins can be used singly or in combination of two or more. As the polyolefin-based resin, polyethylene, polypropylene, or a cyclic polyolefin is preferred, and polyethylene or polypropylene is more preferred, and polypropylene is particularly preferred, from the viewpoint of reducing the discoloration.

As used herein, the expression "made of polyolefin-based resin" means that the polyolefin-based resin is included in at least a portion of the material, and "made of polyolefin-based resin" also includes, for example, a mixture of two or more resins (polymer alloy), which are a polyolefin-based resin and other resins.

A substance that prevents transmission of ultraviolet light, such as an ultraviolet absorber or an ultraviolet scattering agent, is preferably further mixed into the container made of polyolefin-based resin. This improves the photostability of the compound represented by Formula (1). Specific examples of such substances of ultraviolet scattering agents include titanium oxide and zinc oxide, for example. Examples of ultraviolet absorbers include benzotriazole-based ultraviolet absorbers such as 2-(2H-benzotriazol-2-yl)-p-cresol (for example, Tinuvin P: BASF Corporation), 2-(2H-benzotriazol-2-yl)-4,6-bis(1-methyl-1-phenylethyl)phenol (for example, Tinuvin 234: BASF Corporation), 2-(3,5-dit-butyl-2-hydroxyphenyl)benzotriazole (for example, Tinuvin 320: BASF Corporation), 2-[5-chloro(2H)-benzotriazol-2-yl]-4-methyl-6-(tert-butyl)phenol (for example, Tinuvin 326: BASF Corporation), 2-(3,5-di-t-butyl-2-hydroxyphenyl)-5-chlorobenzotriazole (for example, Tinuvin 327: BASF Corporation), 2-(2H-benzotriazol-2-yl)-4,6-di-tert-pentylphenol (for example, Tinuvin PA328: BASF Corporation), 2-(2H-benzotriazol-2-yl)-4-(1,1,3,3-tetramethylbutyl)phenol (for example, Tinuvin 329: BASF Corporation), 2,2'-methylenebis[6-(2H-benzotriazol-2-yl)-4-(1,1,3,3-tetramethylbutyl)phenol (for example, Tinuvin 360: BASF Corporation), a reaction product of methyl 3-(3-(2H-benzotriazol-2-yl)-5-tert-butyl-4-hydroxyphenyl)propionate and polyethylene glycol 300 (for example, Tinuvin 213: BASF Corporation), 2-(2H-benzotriazol-2-yl)-6-dodecyl-4-methylphenol (for example, Tinuvin 571: BASF Corporation), 2-(2'-hydroxy-3',5'-di-t-amylphenyl)benzotriazole, 2-[2'-hydroxy-3'-(3",4",5",6"-tetrahydrophthalimidomethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbers such as 2,2-bis{[2-cyano-3,3-diphenylacryloyloxy]methyl}propane-1,3-diyl=bis(2-cyano-3,3-diphenylacrylate) (for example, Uvinul 3030 FF: BASF Corporation), ethyl 2-cyano-3,3-diphenylacrylate (for example, Uvinul 3035: BASF Corporation), and 2-ethylhexyl 2-cyano-3,3-diphenylacrylate (for example, Uvinul 3039: BASF Corporation); triazine-based ultraviolet absorbers such as 2-(4,6-diphenyl-1,3,5-triazin-2-yl)-5-[(hexyl)oxy]-phenol (for example, Tinuvin 1577 ED: BASF Corporation); benzophenone-based ultraviolet absorbers such as octabenzone (for example, Chimassorb 81: BASF Corporation), 2,2'-dihydroxy-4,4'-dimethoxybenzophenone (for example, Uvinul 3049: BASF Corporation), 2,2'-4,4'-tetrahydrobenzophenone (for example, Uvinul 3050: BASF Corporation), oxybenzone, hydroxymethoxybenzophenonesulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamate-based ultraviolet absorbers such as methyl diisopropylcinnamate, cinoxate, glyceryl mono-2-ethylhexanoate di-p-methoxycinnamate, isopropyl p-methoxycinnamate-diisopropyl cinnamate ester mixture, 2-ethylhexyl p-methoxycinnamate, and benzyl cinnamate; benzoate-based ultraviolet absorbers such as p-aminobenzoic acid, ethyl p-aminobenzoate, glyceryl p-aminobenzoate, amyl p-dimethylaminobenzoate, 2-ethylhexyl p-dimethylaminobenzoate, and ethyl 4-[N,N-di(2-hydroxypropyl)amino]benzoate; salicylate-based ultraviolet absorbers such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino]1,3,5-triazine; p-hydroxyanisole; 4-tert-butyl-4'-methoxydibenzoylmethane; phenylbenzimidazole sulfonate; and hexyl 2-(4-diethylamino-2-hydroxybenzoyl)benzoate.

When a substance that prevents transmission of ultraviolet light is mixed into the container, the proportion of the substance to be incorporated varies depending on the type of the substance and the like, but it may be 0.001 to 50 mass%, for example, preferably 0.002 to 25 mass%, and particularly preferably about 0.01 to 10 mass%, in the container.

The inside of the container is preferably visible (observable) by the naked eye. When the inside is visible, the following advantages are produced. For example, the presence or absence of any foreign matter can be inspected in the manufacturing steps of the pharmaceutical preparation, and the residual amount of the contents (aqueous composition) can be examined by a user of the pharmaceutical preparation. The visibility may be ensured in at least a portion of the surface of the container (for example, even if the side surface of a container for eye drops cannot be seen through due to the presence of a shrinkable film or the like, it can be determined that the inside is visible if the bottom surface of the container is visible). If the inside is visible through a portion of the surface of the container, then the aqueous composition in the container is visible.

The means for storing the aqueous composition into the container is not particularly limited, and the container may be filled with the aqueous composition using a conventional method, in accordance with the form of the container and the like.

The disease targeted by the aqueous composition or the pharmaceutical preparation is not particularly limited, and may be selected as appropriate depending on the pharmacological action or the like of the compound represented by Formula (1).

Specifically, the aqueous composition or pharmaceutical preparation can be used, for example, as a prophylactic or therapeutic agent for ocular hypertension or glaucoma, based on the Rho kinase inhibitory action or intraocular pressure-lowering action of the compound represented by Formula (1), and the intraocular pressure-lowering action of the β blocker. This is preferred because the intraocular pressure-lowering action of the compound represented by Formula (1) and the intraocular pressure-lowering action of the β blocker are combined to produce excellent intraocular pressure-lowering action, which leads to achievement of excellent prophylactic and/or therapeutic action for ocular hypertension or glaucoma. More specifically, examples of types of glaucoma include primary open-angle glaucoma, normal-tension glaucoma, hypersecretion glaucoma, acute closed-angle glaucoma, chronic closed-angle glaucoma, plateau iris syndrome, combined mechanism glaucoma, steroid-induced glaucoma, capsular glaucoma, pigmentary glaucoma, amyloid-associated glaucoma, neovascular glaucoma, and malignant glaucoma.

Further, as disclosed in JP-B-5557408, the pharmaceutical preparation may be used as a prophylactic or therapeutic agent for ocular fundus diseases (lesions that mainly develop in the retina and/or choroidea; specifically, for example, hypertensive or arteriosclerotic ocular fundus abnormalities, central retinal artery occlusion, retinal vein occlusion such as central retinal vein occlusion or branch retinal vein occlusion, diabetic retinopathy, diabetic macular edema, diabetic maculopathy, Eales disease, congenital retinal vascular abnormalities such as Coats disease, von Hippel disease, pulseless disease, macular diseases (such as central serous chorioretinopathy, cystoid macular edema, age-related macular degeneration, macular hole, myopic macular degeneration, vitreoretinal interface maculopathy, drug-related maculopathy, or heredomacular degeneration), retinal detachment (such as rhegmatogenous, tractional and exudative, or the like), retinitis pigmentosa, or retinopathy of prematurity) based on the action of the compound represented by Formula (1). More preferably, the pharmaceutical preparation may be used as a prophylactic or therapeutic agent for diabetic retinopathy, diabetic macular edema, or age-related macular degeneration.

When the aqueous composition or pharmaceutical preparation is used as a prophylactic and/or therapeutic agent for an eye disease (preferably, a disease selected from the group consisting of ocular hypertension, glaucoma, and ocular fundus diseases, particularly preferably a disease selected from the group consisting of ocular hypertension and glaucoma), the aqueous composition or pharmaceutical preparation may be administered about one to three times per day.

### [Examples]

The present invention will be described next in more detail with reference to examples; however, the invention is in no way limited to these examples.

In the following test examples, ripasudil monohydrochloride dihydrate can be produced in accordance with the method described in WO2006/057397, for example.

### [Test Example 1] Preservation test

Aqueous compositions of Example 1 and Comparative Example 1 containing the components in the quantities per 100 mL shown in Table 1 were prepared in accordance with a conventional method, and each stored in a container made of polypropylene.

The resulting aqueous compositions were preserved at 80°C for 1 week. The degree of discoloration (yellowing) after the preservation was evaluated by measuring the color difference (ΔYI) of the aqueous compositions before and after the preservation using a color difference meter (spectrophotometer, CM-700d: Konica Minolta Sensing, Inc.).

The results are shown in Table 1.

**[Table 1]**

| | Example 1 | Comparative Example 1 |
|---|---|---|
| Ripasudil Monohydrochloride Dihydrate | 0.9792 g (0.8 g as the free form) | 0.9792 g (0.8 g as the free form) |
| Carteorol Hydrochloride | 2.0 g | - |
| Anhydrous Sodium Dihydrogen Phosphate | 0.4 g | 0.4 g |
| Glycerin | 2.136 g | 2.136 g |
| Benzalkonium Chloride Concentrated Solution 50 | 0.002 mL | 0.002 mL |
| Sodium Hydroxide | q.s. (pH6.0) | q.s. (pH6.0) |
| Purified Water | Total Amount 100 mL | Total Amount 100 mL |
| ΔYI | 1.7 | 6.0 |

As shown in the results set forth in Table 1, it was revealed that when carteolol is further incorporated in the aqueous composition containing ripasudil, the ΔYI value is significantly lowered in comparison with the case where the aqueous composition does not contain carteolol, and discoloration after high-temperature preservation is reduced.

The foregoing test results revealed that when a β blocker typified by carteolol is further incorporated in the aqueous composition containing the compound represented by Formula (1) typified by ripasudil or a salt thereof or a solvate of the compound or the salt thereof, discoloration (yellowing) is relatively unlikely to occur even after preservation at high temperature, and excellent preservation stability can be achieved.

### [Test Example 2] Preservation test No. 2

Aqueous compositions of Example 2 and Comparative Example 2 containing the components in the quantities per 100 mL shown in Table 2 were prepared in accordance with a conventional method.

Each of the resulting aqueous compositions was preserved at -5°C, during which the presence or absence of crystal precipitation was visually evaluated periodically to determine for which aqueous composition earlier crystal precipitation would be observed. The aqueous composition for which earlier crystal precipitation was not observed was rated as "b", and the aqueous composition for which earlier crystal precipitation was observed was rated as "d".

The results are shown in Table 2.

**[Table 2]**

| | Example 2 | Comparative Example 2 |
|---|---|---|
| Ripasudil Monohydrochloride Dihydrate | 0.9792 g (0.8 g as the free form) | 0.9792 g (0.8 g as the free form) |
| Carteolol Hydrochloride | 2.0 g | - |
| Anhydrous Sodium Dihydrogen Phosphate | 0.4 g | 0.4 g |
| Benzalkonium Chloride Concentrated Solution 50 | 0.002 mL | 0.002 mL |
| Sodium Hydroxide | q.s. (pH6.0) | q.s. (pH6.0) |
| Purified Water | Total Amount 100 mL | Total Amount 100 mL |
| Crystal Precipitation | b | d |

As shown in the results set forth in Table 2, it was revealed that when carteolol is further incorporated in the aqueous composition containing ripasudil, crystal precipitation during low-temperature preservation is suppressed in comparison with the case where the aqueous composition does not contain carteolol.

### [Test Example 3] Preservation test No. 3

Aqueous compositions of Example 3 and Comparative Example 3 containing the components in the quantities per 100 mL shown in Table 3 were prepared in accordance with a conventional method.

Each of the resulting aqueous compositions was preserved at -5°C, during which the presence or absence of crystal precipitation was visually evaluated periodically to determine for which aqueous composition earlier crystal precipitation would be observed. The aqueous composition for which earlier crystal precipitation was not observed was rated as "b", and the aqueous composition for which earlier crystal precipitation was observed was rated as "d".

The results are shown in Table 3.

**[Table 3]**

| | Example 3 | Comparative Example 3 |
|---|---|---|
| Ripasudil Monohydrochloride Dihydrate | 0.9792 g (0.8 g as the free form) | 0.9792 g (0.8 g as the free form) |
| Timolol Maleate | 1.368 g (1.0 g as the free form) | - |
| Anhydrous Sodium Dihydrogen Phosphate | 0.4 g | 0.4 g |
| Benzalkonium Chloride Concentrated Solution 50 | 0.002 mL | 0.002 mL |
| Sodium Hydroxide | q.s. (pH6.0) | q.s. (pH6.0) |
| Purifed Water | Total Amount 100 mL | Total Amount 100 mL |
| Crystal Precipitation | b | d |

As shown in the results set forth in Table 3, it was revealed that when timolol is further incorporated in the aqueous composition containing ripasudil, crystal precipitation during low-temperature preservation is suppressed in comparison with the case where the aqueous composition does not contain timolol.

### [Test Example 4] Preservation test No. 4

Aqueous compositions of Example 4 and Comparative Example 4 containing the components in the quantities per 100 mL shown in Table 4 were prepared in accordance with a conventional method.

Each of the resulting aqueous compositions was preserved at -5°C, during which the presence or absence of crystal precipitation was visually evaluated periodically to determine for which aqueous composition earlier crystal precipitation would be observed. The aqueous composition for which earlier crystal precipitation was not observed was rated as "b", and the aqueous composition for which earlier crystal precipitation was observed was rated as "d".

The results are shown in Table 4.

**[Table 4]**

| | Example 4 | Comparative Example 4 |
|---|---|---|
| Ripasudil Monohydrochloride Dihydrate | 0.9792 g (0.8 g as the free form) | 0.9792 g (0.8 g as the free form) |
| Nipradilol | 0.5 g | - |
| Anhydrous Sodium Dihydrogen Phosphate | 0.4 g | 0.4 g |
| Benzalkonium Chloride Concentrated Solution 50 | 0.002 mL | 0.002 mL |
| Sodium Hydroxide | q.s. (pH6.0) | q.s. (pH6.0) |
| Purified Water | Total Amount 100 mL | Total Amount 100 mL |
| Crystal Precipitation | b | d |

As shown in the results set forth in Table 4, it was revealed that when nipradilol is further incorporated in the aqueous composition containing ripasudil, crystal precipitation during low-temperature preservation is suppressed in comparison with the case where the aqueous composition does not contain nipradilol.

The foregoing results of Test Examples 2 to 4 revealed that when a β blocker typified by carteolol, timolol, and nipradilol is further incorporated in the aqueous composition containing the compound represented by Formula (1) typified by ripasudil or a salt thereof or a solvate of the compound or the salt thereof, precipitation of crystals is relatively unlikely to occur even after preservation at low temperature, and excellent preservation stability can be achieved.

### [Test Example 5] Preservation test No. 5

Aqueous compositions of Examples 5 to 9 containing the components in the quantities per 100 mL shown in Table 5 were prepared in accordance with a conventional method, and each of the aqueous compositions was preserved at -5°C for 1 month, and then visually evaluated for the presence or absence of crystal precipitation. Note that the absence of crystal precipitation was rated as "b", and the presence of crystal precipitation was rated as "d". The results are shown in Table 5.

**[Table 5]**

| | Example 5 | Example 6 | Example 7 | Example 8 | Example 9 |
|---|---|---|---|---|---|
| Ripasudil Monohydrochloride Dihydrate | 0.4896 g | 0.4896 g | 0.4896 g | 0.4896 g | 0.4896 g |
| | (0.4 g as the free form) | (0.4 g as the free form) | (0.4 g as the free form) | (0.4 g as the free form) | (0.4 g as the free form) |
| Timolol Maleate | 0.684 g | 0.684 g | 0.684 g | 0.684 g | 0.684 g |
| | (0.5 g as the free form) | (0.5 gas the free form) | (0.5 g as the free form) | (0.5 g as the free form) | (0.5 g as the free form) |
| L-Glutamic Acid | 0.25 g | - | - | - | - |
| Taurine | - | 0.7 g | - | - | - |
| Monoethanolamine | - | - | 1 g | - | - |
| Epsilon-aminocaproic Acid | - | - | - | 0.5 g | - |
| Trometamol | - | - | - | - | 0.5 g |
| Anhydrous Sodium Dihydrogen Phosphate | 0.4 g | 0.4 g | 0.4 g | 0.4 g | 0.4 g |
| Glycerin | q.s. | q.s. | q.s. | q.s. | q.s. |
| | (osmotic pressure ratio: about 1) | (osmotic pressure ratio: about 1) | (osmotic pressure ratio: about 1) | (osmotic pressure ratio: about1) | (osmotic pressure ratio: about 1) |
| Sodium Hydroxide | q.s. | q.s. | q.s. | q.s. | q.s. |
| | (pH6) | (pH6) | (pH6) | (pH6) | (pH6) |
| Benzalkonium Chloride Concentrated Solution 50 | 0.004 mL | 0.004 mL | 0.004 mL | 0.004 mL | 0.004 mL |
| Purified Water | Balance | Balance | Balance | Balance | Balance |
| Presence or Absence of Crystal Precipitation | b | b | b | b | b |

As shown in the results set forth in Table 5, no crystal precipitation was observed for the aqueous compositions of Examples 5 to 9 even after preservation at -5°C for 1 month.

The foregoing results of Test Example 5 revealed that when an amino compound typified by L-glutamic acid, taurine, monoethanolamine, epsilon-aminocaproic acid, and trometamol is further incorporated in the aqueous composition containing the compound represented by Formula (1) typified by ripasudil or a salt thereof or a solvate of the compound or the salt thereof and a β blocker typified by timolol or a salt thereof or a solvate of timolol or the salt thereof, precipitation of crystals is relatively unlikely to occur even after preservation at low temperature over a long period, and excellent preservation stability can be achieved.

### [Test Example 6] Preservation Test No. 6

An aqueous composition was prepared in the same manner as was the aqueous composition of Example 5 except that 0.005 g of dibutylhydroxytoluene and 0.05 g of polysorbate 80 were used in place of L-glutamic acid, and was preserved at -5°C.

After preservation for 1 month, the presence or absence of crystal precipitation was visually evaluated, and no crystal precipitation was observed.

### [Test Example 7] Preservation test (evaluation of stability of timolol)

Aqueous compositions containing the components in the quantities per 100 mL shown in Table 6 and having a pH value of 4.0, 4.5, 5.0, 5.5, 6.0, 6.5, 7.0, 7.5, or 8.0 at 25°C were prepared in accordance with a conventional method. The pH value of each aqueous composition was adjusted through the amount of hydrochloric acid or sodium hydroxide incorporated.

Each of the resulting aqueous compositions having different pH values was preserved at 60°C for 1 month, and the amount of a degradation product of timolol after the preservation was measured. The amount of a degradation product of timolol was determined as the area percentage (%) relative to the peak area derived from timolol and a degradation product thereof, using an HPLC apparatus.

The results are shown in Table 7.

**[Table 6]**

| Components | Quantity |
|---|---|
| Timolol Maleate | 0.684 g (0.5 g as the free form) |
| Ripasudil Monohydrochloride Dihydrate | 0.4896 g (0.4 g as the free form) |
| Anhydrous Sodium Dihydrogen Phosphate | 0.4 g |
| Glycerin | 1.69 g |
| Benzalkonium Chloride Concentrated Solution 50 | 0.004 mL |
| Hydrochloric Acid/Sodium Hydroxide | q.s. (pH4.0-8.0) |
| Purified Water | Total Amount 100 mL |

**[Table 7]**

| | Amount of Degradation Product of Timolol (%) | | | | | | | | |
|---|---|---|---|---|---|---|---|---|---|
| pH | 4.0 | 4.5 | 5.0 | 5.5 | 6.0 | 6.5 | 7.0 | 7.5 | 8.0 |
| After Preservation at 60°C for 1 Month | 0.0 | 0.0 | 0.0 | 0.0 | 0.1 | 0.3 | 0.6 | 1.2 | 2.1 |

As shown in the results set forth in Table 7, it was revealed that, in the aqueous composition containing ripasudil and timolol, timolol is stable in an environment of pH 6.5 or lower, and the amount of the degradation product decreases as the pH value is lowered until the pH reaches 5.5, and in contrast timolol is unstable over pH 6.5, and the amount of the degradation product increases as the pH value is elevated.

The pH value of an aqueous composition containing timolol singly (eye drop) is typically set to around 7.0 (for example, Timoptol eye drop: Santen Pharmaceutical Co., Ltd., RYSMON Ophthalmic Solution: Wakamoto Pharmaceutical Co., Ltd., and TIMOLATE Ophthalmic Solution: Nitten Pharmaceutical Co., Ltd.). However, it was revealed that it is preferred to set the pH value of the aqueous composition containing ripasudil and timolol to a pH value lower than around 7.0 from the viewpoint of the stability of timolol.

The foregoing test results revealed that when the pH value of the aqueous composition containing the compound represented by Formula (1) typified by ripasudil or a salt thereof or a solvate of the compound or the salt thereof and timolol or a salt thereof or a solvate of timolol or the salt thereof at 25°C is set to 6.5 or lower, timolol or a salt thereof or a solvate of timolol or the salt thereof can be stabilized.

### [Test Example 8] Preservation test (evaluation of stability of ripasudil)

Aqueous compositions containing the components in the quantities per 100 mL shown in Table 6 and having a pH value of 4.0, 4.5, 5.0, 5.5, 6.0, or 6.5 at 25°C were prepared in the same manner as in Test Example 7.

Each of the resulting aqueous compositions having different pH values was preserved at 60°C for 1 month, and the amount of a degradation product of ripasudil after the preservation was measured. The amount of a degradation product of ripasudil was determined as the area percentage (%) relative to the peak area derived from ripasudil and a degradation product thereof, using an HPLC apparatus.

The results are shown in Table 8.

**[Table 8]**

| | Amount of Degradation Product of Ripasudil (%) | | | | | |
|---|---|---|---|---|---|---|
| pH | 4.0 | 4.5 | 5.0 | 5.5 | 6.0 | 6.5 |
| After Preservation at 60°C for 1 Month | 1.9 | 2.4 | 3.6 | 7.0 | 10.3 | 11.4 |

As shown in the results set forth in Table 8, it was revealed that, in the aqueous composition containing ripasudil and timolol and having a pH value of 6.5 or lower, the amount of a degradation product of ripasudil decreases as the pH value is lowered.

The foregoing test results revealed that when the pH value of the aqueous composition containing the compound represented by Formula (1) typified by ripasudil or a salt thereof or a solvate of the compound or the salt thereof and timolol or a salt thereof or a solvate of timolol or the salt thereof and having a pH value of 6.5 or lower is further lowered, the compound represented by Formula (1) or a salt thereof or a solvate of the compound or the salt thereof can be also stabilized, and that when the pH value at 25°C is set to 5.5 or lower (preferably 5.0 or lower, more preferably 4.5 or lower), for example, both of the components can be stabilized.

### [Production Examples 1 to 81]

Aqueous compositions containing the components in the quantities (amounts (g) per 100 mL of the aqueous composition) shown in Tables 9 to 17 can be produced in accordance with a conventional method.

**[Table 9]**

| | Production Example 1 | Production Example 2 | Production Example 3 | Production Example 4 | Production Example 5 | Production Example 6 | Production Example 7 | Production Example 8 | Production Example 9 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Carteolol Hydrochloride (as the amount of hydrochloride) | 1 | 2 | - | - | - | 2 | - | - | - |
| Nipradilol | - | - | 0.25 | - | - | - | 0.25 | | - |
| Timolol Maleate (as the amount of the free form) | - | - | - | 0.25 | 0.5 | - | - | 0.25 | 0.5 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | - | - | - | - | - | - | - | - | - |
| Borax | - | - | - | - | - | - | - | - | - |
| Sodium Dihydrogenphosphate Monohydrate | 0.4 | 0.4 | 0.4 | - | - | 0.4 | 0.4 | 0.4 | 0.4 |
| Dibasic Sodium Phosphate Hydrate | - | - | - | - | - | - | - | q.s. | q.s. |
| Anhydrous Sodium Monohydrogen Phosphate | - | - | - | - | - | q.s. | q.s. | - | - |
| Potassium Dihydrogenphosphate | - | - | - | 0.4 | 0.4 | - | - | - | - |
| Sodium Hydroxide | q.s. | q.s. | q.s. | q.s. | - | - | - | - | - |
| Trometamol | - | - | - | - | - | - | - | - | - |
| Hydrochloric Acid | - | - | - | - | - | - | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount 100 mL | Total Amount | Total Amount 100 mL | Total Amount 100 mL |
| pH | 5 | 5 | 6 | 6 | 6.5 | 6.5 | 4.5 | 4.5 | 4 |

**[Table 10]**

| | Production Example 10 | Production Example 11 | Production Example 12 | Production Example 13 | Production Example 14 | Production Example 15 | Production Example 16 | Production Example 17 | Production Example 18 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Carteolol Hydrochloride (as the amount of hydrochloride) | 1 | 2 | - | - | - | 2 | - | - | - |
| Nipradifol | - | - | 0.25 | - | - | - | 0.25 | - | - |
| Timolol Maleate (as the amount of the free form) | - | - | - | 0.25 | 0.5 | - | - | 0.25 | 0.5 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | 1.0 | 1.0 | 1.0 | 1.0 | 1.0 | 1.0 | 1.0 | 1.0 | 1.0 |
| Borax | - | - | - | - | q.s. | q.s. | - | - | - |
| Sodium Dihydrogenphosphate Monohydrate | - | - | - | - | - | - | - | - | - |
| Dibasic Sodium Phosphate Hydrate | - | - | - | - | - | - | - | - | - |
| Anhydrous Sodium Monohydrogen Phosphate | - | - | - | - | - | - | - | - | - |
| Potassium Dihydrogenphosphate | - | - | - | - | - | - | - | - | - |
| Sodium Hydroxide | q.s. | q.s. | q.s. | q.s. | - | - | - | - | - |
| Trametamol | - | - | - | - | - | - | - | - | - |
| Hydrochloric Acid | - | - | - | - | - | - | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | | | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 4.5 | 4.5 | 4 |

**[Table 11]**

| | Production Example 19 | Production Example 20 | Production Example 21 | Production Example 22 | Production Example 23 | Production Example 24 | Production Example 25 | Production Example 26 | Production Example 27 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 08 | 0.8 |
| Carteolol Hydrochloride (as the amount of hydrochloride) | 1 | 2 | - | - | - | 2 | - | - | - |
| Nipradilol | - | - | 0.25 | - | - | - | 0.25 | - | - |
| Timolol Maleate (as the amount of the free form) | - | - | - | 0.25 | 0.5 | - | - | 0.25 | 0.5 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | - | - | - | - | - | - | - | - | - |
| Borax | - | - | - | - | - | - | - | - | - |
| Sodium Dihydrogenphosphate Monohydrate | - | - | - | - | - | - | - | - | - |
| Dibasic Sodium Phosphate Hydrate | - | - | - | - | - | - | - | - | - |
| Anhydrous Sodium Monohydragen Phosphate | - | - | - | - | - | - | - | - | - |
| Potassium Dihydrogenphosphate | - | - | - | - | - | - | - | - | - |
| Sodium Hydroxide | - | - | - | - | - | - | - | - | - |
| Trometamol | 1.5 | 1.5 | 1.5 | 1.5 | 1.5 | 1.5 | 1.5 | 1.5 | 1.5 |
| Hydrochloric Acid | q.s. | q.s. | q.s. | q.s. | q.s. | q.s. | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | | | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 4.5 | 4.5 | 4 |

**[Table 12]**

| | Production Example 28 | Production Example 29 | Production Example 30 | Production Example 31 | Production Example 32 | Production Example 33 | Production Example 34 | Production Example 35 | Production Example 36 |
|---|---|---|---|---|---|---|---|---|---|
| Ripasudil Monohydrochloride Dihydrate (as the amount of the free form) | 0.4 | 0.4 | 0.4 | 0.4 | 0.4 | 0.4 | 0.4 | 0.4 | 0.4 |
| Timolol Maleate | 0.25 | 0.25 | 0.25 | 0.25 | 0.25 | 0.25 | 0.25 | 0.25 | 0.25 |
| L-Glutamic Acid | 0.25 | - | - | - | - | - | - | - | 0.1 |
| Taurine | - | - | - | - | - | - | - | 0.2 | - |
| Monoethanolamine | 0.2 | - | 0.3 | 0.5 | - | - | 1 | - | - |
| Epsilon-aminocaproic Acid | - | - | 0.1 | - | - | 0.2 | - | - | - |
| Trometamol | - | 3 | - | - | 0.3 | - | 1 | - | 15 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Sodium Dihydrogen phosphate Monohydrate | 0.4 | 0.4 | 0.4 | - | - | 0.4 | 0.4 | 0.4 | 0.4 |
| Dibasic Sodium Phosphate Hydrate | - | - | - | - | - | - | - | q.s. | q.s. |
| Anhydrous Sodium Monohydrogen Phosphate | - | - | - | - | - | q.s. | q.s. | - | - |
| Potassium Dihydrogenphosphate | - | - | - | 0.4 | 0.4 | - | - | - | - |
| Sodium Hydroxide | q.s. | q.s. | q.s. | q.s. | q.s. | - | - | - | - |
| D-Mannitol | 0.5 | - | 1 | - | 2 | - | 4 | - | - |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 7 | 7 | 8 |

**[Table 13]**

| | Production Example 37 | Production Example 38 | Production Example 39 | Production Example 40 | Production Example 41 | Production Example 42 | Production Example 43 | Production Example 44 | Production Example 45 |
|---|---|---|---|---|---|---|---|---|---|
| Ripasudil Monohydrochloride Dihydrate (as the amount of the free form) | 0.4 | 0.4 | 0.4 | 0.4 | 0.4 | 0.4 | 0.4 | 0.4 | 0.4 |
| Timolol Maleate | 0.5 | 0.5 | 0.5 | 0.5 | 0.5 | 0.5 | 0.5 | 0.5 | 0.5 |
| L-Glutamic Acid | - | - | - | - | 0.1 | 0.25 | - | - | - |
| Taurine | - | - | - | 0.2 | - | - | - | - | - |
| Monoethanolamine | - | - | 1 | - | - | 0.2 | - | 0.3 | 0.5 |
| Epsilon-aminocaproic Acid | - | 0.2 | - | - | - | - | - | 0.1 | - |
| Trometamol | 0.3 | - | 1 | - | 15 | - | 3 | - | - |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | 1.0 | 1.0 | 1.0 | 1.0 | 1.0 | 1.0 | 1.0 | 1.0 | 1.0 |
| Borax | - | - | - | - | q.s. | q.s. | q.s. | q.s. | q.s. |
| Sodium Hydroxide | q.s. | q.s. | q.s. | q.s. | - | - | - | - | - |
| D-Mannitol | - | 0.5 | - | 1 | - | 2 | - | 4 | - |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01. | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100mL | Total Amount 100 mL | Total Amount 100 mL | Total Amount 100 mL | Total Amount 100 mL |
| pH | 5 | 5 | 6 | 6 | 6.5 | 6.5 | 7 | 7 | 8 |

**[Table 14]**

| | Production Example 46 | Production Example 47 | Production Example 48 | Production Example 49 | Production Example 50 | Production Example 51 | Production Example 52 | Production Example 53 | Production Example 54 |
|---|---|---|---|---|---|---|---|---|---|
| Ripasudil Monohydrochloride Dihydrate (as the amount of the free form) | 0.4 | 0.4 | 0.4 | 0.4 | 0.4 | 0.4 | 0.4 | 0.4 | 0.4 |
| Timolol | 0.25 | 0.5 | 0.25 | 0.5 | 0.25 | 0.5 | 0.25 | 0.5 | 0.25 |
| L-Glutamic Acid | 0.25 | - | - | - | - | - | - | - | 0.1 |
| Taurine | - | - | - | - | - | - | - | 0.2 | - |
| Monoethanolamine | 0.2 | - | 0.3 | 0.5 | - | - | 1 | - | - |
| Epsilon-aminocaproic Acid | - | - | 0.1 | - | - | 0.2 | - | - | - |
| Trometamol | - | 3 | - | - | 0.3 | - | 1 | - | 15 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| D-Mannitol | - | - | 0.5 | - | 1 | - | 2 | - | 4 |
| Hydrochloric Acid | q.s. | q.s. | q.s. | q.s. | q.s. | q.s. | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount mL | Total Amount 100 mL | Total Amount 100 | Total Amount 100 |
| pH | 5 | 5 | 6 | 6 | 6.5 | 6.5 | 7 | 7 | 8 |

**[Table 15]**

| | Production Example 55 | Production Example 56 | Production Example 57 | Production Example 58 | Production Example 59 | Production Example 60 | Production Example 61 | Production Example 62 | Production Example 63 |
|---|---|---|---|---|---|---|---|---|---|
| Ripasudil Monohydrochloride Dehydrate (as the amount of the free form) | 0.2 | 0.2 | 0.2 | 0.4 | 0.4 | 0.4 | 0.8 | 0.8 | 0.8 |
| Timolol Maleate (as the amount of the free form) | 0.25 | 0.25 | 0.25 | 0.25 | 0.25 | 0.25 | 0.25 | 0.25 | 0.25 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | - | - | - | - | - | - | - | - | - |
| Borax | - | - | - | - | - | - | - | - | - |
| Sodium Dihydrogenphosphate Monohydrate | 0.4 | 0.4 | 0.4 | - | - | 0.4 | 0.4 | 0.4 | 0.4 |
| Dibasic Sodium Phosphate Hydrate | - | - | - | - | - | - | - | q.s. | q.s. |
| Anhydrous Sodium Monohydrogen Phosphate | - | - | - | - | - | q.s. | q.s. | - | - |
| Potassium Dihydrogenphosphate | - | - | - | 0.4 | 0.4 | - | - | - | - |
| Sodium Hydroxide | q.s. | q.s. | q.s. | q.s. | q.s. | - | - | - | - |
| Trometamol | - | - | - | - | - | - | - | - | - |
| Hydrochloric Acid | - | - | - | - | - | - | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 4.5 | 4 | 3.5 |

**[Table 16]**

| | Production Example 64 | Production Example 65 | Production Example 66 | Production Example 67 | Production Example 68 | Production Example 69 | Production Example 70 | Production Example 71 | Production Example 72 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Timolol Maleate (as the amount of the free form) | 0.5 | 0.5 | 0.5 | 0.5 | 0.5 | 0.5 | 0.5 | 0.5 | 0.5 |
| Sodium chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | 1.0 | 1.0 | 1.0 | 1.0 | 1.0 | 1.0 | 1.0 | 1.0 | 1.0 |
| Borax | - | - | - | - | q.s. | q.s. | - | - | - |
| Sodium Dihydrogenphosphate Monohydrate | - | - | - | - | - | - | - | - | - |
| Dibasic Sodium Phosphate Hydrate | - | - | - | - | - | - | - | - | - |
| Anhydrous Sodium Monohydrogen Phosphate | - | - | - | - | - | - | - | - | - |
| Potassium Dihydrogenphosphate | - | - | - | - | - | - | - | - | - |
| Sodium Hydroxide | q.s. | q.s. | q.s. | q.s. | - | - | - | - | - |
| Trometamol | - | - | - | - | - | - | - | - | - |
| Hydrochloric Acid | - | - | - | - | - | - | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 4.5 | 4 | 3 |

**[Table 17]**

| | Production Example 73 | Production Example 74 | Production Example 75 | Production Example 76 | Production Example 77 | Production Example 78 | Production Example 79 | Production Example 80 | Production Example 81 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Timolol | 0.25 | 0.5 | 0.25 | 0.5 | 0.25 | 0.5 | 0.25 | 0.5 | 0.25 |
| Sodium Chloride | 0.65 | - | - | - | 0.3 | 0.3 | 0.3 | 0.3 | - |
| Glycerin | - | 2 | - | - | 1 | - | - | 0.5 | 1 |
| Propylene Glycol | - | - | 2 | - | - | 1 | - | 0.5 | 1 |
| Potassium Chloride | - | - | - | 0.6 | - | - | 0.3 | - | - |
| Boric Acid | - | - | - | - | - | - | - | - | - |
| Borax | - | - | - | - | - | - | - | - | - |
| Sodium Dihydrogenphosphate Monohydrate | - | - | - | - | - | - | - | - | - |
| Dibasic Sodium Phosphate Hydrate | - | - | - | - | - | - | - | - | - |
| Anhydrous Sodium Monohydrogen Phosphate | - | - | - | - | - | - | - | - | - |
| Potassium Dihydrogenphosphate | - | - | - | - | - | - | - | - | - |
| Sodium Hydroxide | - | - | - | - | - | - | - | - | - |
| Trometamol | 1.5 | 1.5 | 1.5 | 1.5 | 1.5 | 1.5 | 1.5 | 1.5 | 1.5 |
| Hydrochloric Acid | q.s. | q.s. | q.s. | q.s. | q.s. | q.s. | q.s. | q.s. | q.s. |
| Citric Acid Hydrate | 0.1 | - | - | - | - | 0.1 | - | - | - |
| Sodium Acetate Hydrate | - | 0.1 | - | - | - | 0.1 | - | - | - |
| Sodium Edetate | - | - | - | 0.1 | - | - | 0.1 | - | - |
| Benzalkonium Chloride | 0.001 | 0.005 | - | 0.001 | 0.005 | 0.01 | 0.001 | 0.005 | - |
| Benzethonium Chloride | - | - | - | - | - | - | - | - | 0.01 |
| Methyl Parahydroxybenzoate | - | - | 0.01 | - | - | - | 0.01 | - | - |
| Propyl Parahydroxybenzoate | - | - | 0.01 | - | - | - | 0.01 | - | - |
| Chlorobutanol | - | - | - | 0.2 | - | - | - | 0.2 | - |
| Polysorbate 80 | 0.3 | - | - | 0.3 | 0.3 | - | - | 0.3 | 0.3 |
| Polyoxyethylene Castor Oil 60 | - | 0.3 | - | 0.3 | - | 0.3 | - | 0.3 | 0.3 |
| Polyethylene Glycol Monostearate | - | - | 1.5 | 1.5 | - | - | 1.5 | - | 1.5 |
| Purified Water Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 4.5 | 4 | I 3.5 |

### [Production Examples 82 to 162]

Aqueous compositions of Production Examples 82 to 162 can be produced in accordance with a conventional method as in Production Examples 1 to 81, except that instead of ripasudil monohydrochloride dihydrate, an equal amount of 4-bromo-5-{[(2S)-2-methyl-1,4-diazepan-1-yl]sulfonyl}isoquinoline is used.

### [Production Examples 163 to 216]

Pharmaceutical preparations of Production Examples 163 to 216 can be produced in accordance with a conventional method by storing the aqueous compositions of Production Examples 1 to 54 in a container made of polypropylene for eye drops.

### [Production Examples 217 to 270]

Pharmaceutical preparations of Production Examples 217 to 270 can be produced in accordance with a conventional method by storing the aqueous compositions of Production Examples 1 to 54 in a container made of polyethylene for eye drops.

### [Industrial Applicability]

In accordance with the present invention, aqueous compositions having excellent preservation stability can be provided, which can be advantageously used in the pharmaceutical industry, for example.

## Claims

1. An aqueous composition comprising a compound represented by Formula (1): 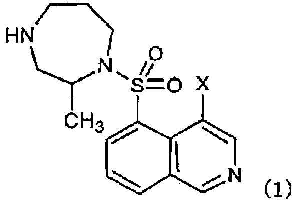 wherein X represents a halogen atom,
or a salt thereof, or a solvate of the compound or the salt thereof, and a β blocker.

2. The aqueous composition according to claim 1, wherein
the compound represented by Formula (1) is ripasudil.

3. The aqueous composition according to claim 1 or 2, wherein the β blocker is one or more selected from the group consisting of carteolol, timolol, nipradilol, a salt of carteolol, a salt of timolol, a salt of nipradilol, a solvate of carteolol or the salt thereof, a solvate of timolol or the salt thereof, and a solvate of nipradilol or the salt thereof.

4. A pharmaceutical preparation obtained by storing the aqueous composition according to any one of claims 1 to 3 in a container made of polyolefin-based resin.

5. The pharmaceutical preparation according to claim 4, wherein the polyolefin-based resin is polypropylene.

6. A method for reducing discoloration of an aqueous composition, the method comprising the step of incorporating a β blocker in an aqueous composition comprising a compound represented by Formula (1): 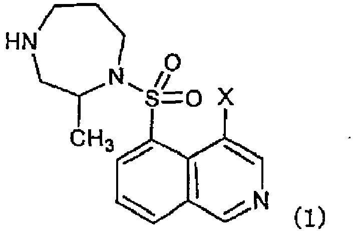 wherein X represents a halogen atom,
or a salt thereof, or a solvate of the compound or the salt thereof.

7. The method according to claim 6, wherein
the compound represented by Formula (1) is ripasudil.

8. The method according to claim 6 or 7, wherein the β blocker is one or more selected from the group consisting of carteolol, timolol, nipradilol, a salt of carteolol, a salt of timolol, a salt of nipradilol, a solvate of carteolol or the salt thereof, a solvate of timolol or the salt thereof, and a solvate of nipradilol or the salt thereof.
